# FASCICULE DE BREVET EUROPEEN

(11) **EP 3 568 166 B1**
(45) Date de publication et mention de la délivrance du brevet: **09.06.2021**
(21) Numéro de dépôt: 18700301.7
(22) Date de dépôt: 15.01.2018
(51) Int. Cl.: A61L 27/12, A61L 27/36, A61L 27/56

(54) **MATERIAU DE REGENERATION OSSEUSE**
KNOCHENREGENERATIONSMATERIAL
BONE REGENERATION MATERIAL

(30) Priorité: 16.01.2017 BE 175025; 16.01.2017 WO PCT/EP2017/050779
(43) Date de publication de la demande: 20.11.2019
(73) Titulaire: Wishbone, 4400 Flemalle5 (BE)
(72) Inventeur: DORY, Emilie, 5300 Andenne (BE); ROMPEN, Eric, 4020 Liège (BE); LAMBERT, France, 4870 Trooz (BE); LECLOUX, Geoffrey, 4050 Chaudfontaine (BE)
(74) Mandataire: Calysta NV
(86) Numéro de dépôt international: PCT/EP2018/050877
(87) Numéro de publication internationale: WO 2018/130686

(56) Documents cités:
- EP-A2- 0 278 583
- JP-A- 2000 327 314

## Description

La présente invention se rapporte à un matériau de régénération osseuse comprenant une première phase solide d'hydroxyapatite d'origine naturelle macroporeuse présentant des pores de diamètres supérieurs ou égaux à 50 µm, de préférence des pores de diamètres compris entre 50 et 100 µm.

Un tel matériau de régénération osseuse est connu du document WO2015/049336 et est utilisé dans le cadre du traitement de détériorations osseuses dans différents domaines comme la chirurgie réparatrice ou esthétique.

L'hydroxyapatite est une espèce minérale de la famille des phosphates, de formule Ca₅(PO₄)₃(OH). Plus spécifiquement, l'hydroxyapatite appartient à la famille cristallographique des apatites qui sont des composés isomorphes possédant une même structure hexagonale. Ce composé est largement utilisé comme biomatériau depuis de nombreuses années dans différentes spécialités médicales car les hydroxyapatites sont les phosphates de calcium cristallins les plus fréquents et les premiers constituants minéraux des os, de l'émail dentaire et de la dentine. Par ailleurs, les hydroxyapatites (et en particulier les hydroxyapatites d'origine naturelle) présentent de bonnes propriétés de biocompatibilité et d'adsorption spécifique des cellules ou des protéines.

Il est ainsi reconnu que l'hydroxyapatite d'origine naturelle présente des propriétés ostéoconductrices ainsi qu'une structure cristalline et une morphologie identiques à celles d'un matériau osseux naturel chez l'humain, l'hydroxyapatite convenant par conséquent parfaitement dans le cadre d'une chirurgie reconstructrice ou réparatrice de l'os. En ce sens, de nombreux matériaux comprenant de l'hydroxyapatite sont actuellement utilisés comme implants dentaires pour stimuler la reconstruction osseuse sur des sites osseux présentant des défauts ou des détériorations. En particulier, l'utilisation d'une hydroxyapatite naturelle provenant d'échantillons osseux nettoyés de leurs substances organiques (protéines, prions, peptides et lipides) permet d'obtenir une matrice particulièrement propice à la régénération osseuse en comparaison avec son analogue artificiel. Il est préférable que le matériau de régénération osseuse soit préalablement épuré de toute trace de substances organiques de sorte que l'implant soit bien accepté/intégré par l'organisme, soit biocompatible et puisse interagir par ostéoconduction avec l'environnement biologique dans lequel il est placé.

A titre d'exemples, les hydroxyapatites peuvent servir de matériaux de substitution pour le remplacement ou la régénération de tissus malades ou endommagés. Elles sont également fréquemment utilisées comme revêtement (coating) sur des prothèses en titane pour faciliter l'ostéointégration ou encore pour empêcher l'usure due à des micromouvements entre l'implant et l'os. Les hydroxyapatites peuvent également être utilisées en tant que ciment afin de remplacer les greffes osseuses autogènes. En outre, un nombre croissant d'applications de l'hydroxyapatite en tant que vecteur de médicaments se développe, ceci parce que l'hydroxyapatite présente une structure avec des micropores interconnectés.

Comme mentionné ci-dessus, l'hydroxyapatite possède une composition chimique très proche de celle de la phase minérale de l'os et ses propriétés biologiques ainsi que sa biocompatibilité en font un excellent produit de substitution osseuse. Précisons qu'une colonisation osseuse dépend des caractéristiques poreuses du matériau de régénération osseuse et de l'interconnexion entre ses macropores (nombre et dimension). Ces interconnexions constituent des sortes de « tunnels » qui permettent le passage des cellules et la circulation sanguine entre les pores favorisant ainsi la formation osseuse.

L'hydroxyapatite, de par son haut niveau de biocompatibilité et sa lente résorption dans l'organisme, peut être administrée sous différentes formes et dans de nombreux tissus pour des applications diverses. On distingue notamment des ciments malléables à base d'hydroxyapatite qui sont préparés puis administrés sur la zone à traiter où ils durcissent. Ces ciments sont utilisés en tant que substitut osseux du fait de leur bonne ostéointégration et de leur biorésorbabilité permettant de laisser place à un os néoformé au cours du temps.

Il existe également des solutions et des gels à base d'hydroxyapatite pouvant contenir un polymère comme de la carboxyméthylcellulose. On parle alors d'implants composites céramiquepolymère bioactifs, ces produits pouvant être utilisés dans différents domaines comme pour combler des déficiences parodontales en chirurgie dentaire ou faire du comblement en chirurgie orthopédique.

Certains produits à base d'hydroxyapatite se présentent encore sous forme de bloc, de poudre ou de granulés utilisés en orthopédie, en comblement ou en tant que complément aux implants dentaires.

Parmi les produits à base d'hydroxyapatite, nombreux sont ceux qui ne sont pas prêts à l'emploi et qui nécessitent une préparation préalable. C'est le cas notamment des ciments à base d'hydroxyapatite qui requièrent un mélange préalable d'une poudre et d'une solution avant dépôt sur ou dans une zone à traiter où un durcissement in situ s'opère alors. D'autres produits à base d'hydroxyapatite nécessitent encore d'être mis en forme avant implantation sur ou dans la zone à traiter.

Malheureusement, même si un tel matériau de régénération osseuse comprenant une phase solide d'hydroxyapatite d'origine naturelle macroporeuse présente des propriétés propices à une implantation endoosseuse et permet une régénération osseuse correcte grâce à sa biocompatibilité et à ses capacités ostéoconductrices, il est observé en pratique que ce type de matériau de régénération osseuse n'est pas toujours optimal et que le délai du processus de régénération osseuse est toujours relativement long (1 mm/mois). Par ailleurs, la quantité de régénération osseuse est fréquemment insuffisante, ce qui entraîne la formation de fibroses partielles plutôt qu'une ossification totale.

Dans un but d'accélération du processus de régénération osseuse, le document US2002/0114755 divulgue un matériau comprenant de l'hydroxyapatite et 50 à 90% en masse de phosphate tricalcique, le matériau selon ce document antérieur étant obtenu par conversion de tissus d'algues dures dans une solution aqueuse de phosphate avec l'addition d'ions Mg⁺⁺ à température élevée.

Le matériau de régénération osseuse selon ce document antérieur, c'est-à-dire qui comprend de l'hydroxyapatite et 50 à 90% en masse de phosphate tricalcique d'origine naturelle, présente une structure poreuse interconnectée, une résorption améliorée et contrôlable dans l'organisme conduisant à une régénération osseuse accélérée.

Malheureusement, même si un matériau de régénération osseuse selon le document US2002/0114755 permet d'accélérer le processus de régénération osseuse en augmentant l'apport de calcium et de phosphore sous forme d'ions libres dans l'environnement direct du site d'implantation, il est observé que ce type de matériau n'est pas optimal.

En effet, le procédé d'obtention d'un tel matériau de régénération osseuse consiste en la transformation d'hydroxyapatite en phosphate tricalcique. Il s'agit donc de la conversion d'une première phase naturelle en une seconde phase naturelle, l'hydroxyapatite étant remplacé par le phosphate tricalcique.

Ainsi, lors de l'implantation du matériau de régénération osseuse selon le document US2002/0114755, le phosphate tricalcique qui est plus soluble que l'hydroxyapatite va rapidement se solubiliser et libérer des ions calcium et phosphore dans l'environnement du site d'implantation. Comme cité ci-dessus, le phosphate tricalcique d'un tel matériau de régénération osseuse représente 50 à 90% en masse et est obtenu par la conversion d'une partie de l'hydroxyapatite contenue dans le matériau de départ, à savoir du tissu d'algues dures. Par conséquent, avec un matériau de régénération osseuse selon ce document antérieur, 50 à 90% de la masse (correspondant au phosphate tricalcique) va rapidement se solubiliser suite à l'implantation et conduire ainsi à un matériau d'hydroxyapatite naturel présentant de larges zones de vide et donc à un matériau d'hydroxyapatite qui est certes d'origine naturelle mais qui ne présente plus les caractéristiques topographiques (porosité, surface spécifique,...) optimales permettant une bonne régénération osseuse.

Un matériau de régénération osseuse selon le document US2002/0114755 ne permet donc pas de maintenir sur le long terme la structure microporeuse adéquate de l'hydroxyapatite naturelle une fois celui-ci implanté et ne permet donc pas d'assurer un processus de régénération osseuse optimal au site d'implantation en termes de prolifération et de différenciation des cellules osseuses car un tel matériau, une fois implanté, ne possède plus une topographie superficielle ni une microporosité adéquate.

Le document EP 0278583A2 divulgue un matériau de remplacement osseux comprenant de l'hydroxyapatite provenant d'os naturel, ayant des pores de diamètre compris entre 5 et 500 µm. Le matériau comprend deux phases dont une ayant un ratio molaire Ca/P compris entre 1,0 and 2,0. La seconde phase comprend du tetracalcium de phosphate. Le document divulgue aussi un procédé de fabrication qui consiste à tremper l'os naturel dans une solution de phosphore ou dans une solution comprenant du calcium suivi d'un étape de frittage.

L'invention a pour but de pallier les inconvénients de l'état de la technique en procurant un matériau de régénération osseuse permettant de minimiser le temps de régénération osseuse tout en stimulant le potentiel de régénération osseuse de telle sorte à obtenir une quantité d'os régénéré optimale.

Le matériau de régénération osseuse selon l'invention présente non seulement des propriétés chimiques (relargage optimisé en calcium et en phosphore sous forme d'ions libres) qui permettent de minimiser le temps de régénération osseuse, mais présente également des propriétés physiques (topographie et porosité conservées) optimales autorisant une bonne colonisation osseuse de telle sorte à obtenir une quantité d'os régénéré optimale.

Pour résoudre ce problème, il est prévu suivant l'invention, un matériau de régénération osseuse comprenant une première phase solide d'hydroxyapatite d'origine naturelle macroporeuse présentant des pores de diamètres supérieurs ou égaux à 50 µm, de préférence des pores de diamètres compris entre 50 et 100 µm, ledit matériau de régénération osseuse étant caractérisé en ce que ladite première phase solide d'hydroxyapatite est enrichie par une deuxième phase de phosphate de calcium, ladite deuxième phase étant une phase synthétique solide, présentant un ratio molaire Ca/P compris entre 0,2 et 2, de préférence compris entre 0,3 et 1,8, préférentiellement compris entre 0,5 et 1,65, et présentant un produit de solubilité Ks supérieur à celui de ladite première phase d'hydroxyapatite d'origine naturelle, ledit matériau de régénération osseuse présentant un ratio en poids défini entre ladite première phase solide d'hydroxyapatite d'origine naturelle et ladite deuxième phase synthétique solide de phosphate de calcium compris entre 99/1 et 1/99.

Avantageusement, selon l'invention, le ratio en poids défini entre ladite première phase solide d'hydroxyapatite d'origine naturelle et ladite deuxième phase synthétique solide de phosphate de calcium est de 95/5, 90/10, 85/15, 80/20, 75/25, 70/30, 65/35, 60/40, 55/45, 50/50, 45/55, 40/60, 35/65, 30/70, 25/75, 20/80, 15/85, 10/90 ou 5/95.

Dans le cadre de la présente invention, il a été mis en évidence qu'un tel matériau de régénération osseuse suivant l'invention présentant :
- une première phase solide d'hydroxyapatite d'origine naturelle présentant des pores de diamètres supérieurs ou égaux à 50 µm, de préférence des pores de diamètres compris entre 50 et 100 µm ;
- une deuxième phase synthétique solide de phosphate de calcium destinée à enrichir ladite première phase ;
- un ratio molaire Ca/P pour la deuxième phase synthétique solide de phosphate de calcium compris entre 0,2 et 2, de préférence compris entre 0,3 et 1,8, préférentiellement compris entre 0,5 et 1,65 et présentant un produit de solubilité Ks supérieur à celui de ladite première phase d'hydroxyapatite d'origine naturelle; et
- un ratio en poids défini entre ladite première phase solide d'hydroxyapatite d'origine naturelle et ladite deuxième phase synthétique solide de phosphate de calcium compris entre 99/1 et 1/99, permet d'assurer un relargage adéquat de calcium (par exemple sous la forme d'ions libres extracellulaires Ca²⁺) et phosphore (par exemple sous la forme d'ions libres extracellulaires PO₄³⁻) dans l'environnement du site de régénération osseuse de telle sorte que ceux-ci puissent jouer le rôle de promoteurs de la repousse des tissus biologiques environnants en favorisant significativement la prolifération et la différentiation des cellules osseuses ainsi que la minéralisation.

En outre, il a été observé de manière surprenante que le matériau de régénération osseuse selon l'invention, c'est-à-dire dont la phase solide d'hydroxyapatite est une phase d'hydroxyapatite naturelle qui est enrichie par une deuxième phase de phosphate de calcium, présente des caractéristiques topographiques (porosité, surface spécifique, ...) optimales permettant d'optimiser la régénération osseuse en autorisant une bonne colonisation osseuse, et ce même après l'implantation du matériau de régénération osseuse.

Une bonne colonisation osseuse dépend des caractéristiques poreuses du matériau de régénération osseuse et de l'interconnexion entre ses macropores (nombre et dimensions), ces interconnexions constituant des sortes de « tunnels » qui permettent le passage des cellules et la circulation sanguine entre les pores favorisant la formation osseuse. La conservation des caractéristiques topographiques de l'hydroxyapatite naturelle est donc capitale pour obtenir une régénération osseuse adéquate, ce qui n'est pas le cas avec un matériau de régénération selon le document US2002/0114755 où une partie (50 à 90% en masse) de la phase naturelle d'hydroxyapatite est convertie en une phase naturelle de phosphate tricalcique.

Un matériau de régénération osseuse selon l'invention présente donc à la fois des propriétés chimiques et physiques optimales (relargage optimisé en calcium et en phosphore sous forme d'ions libres / topographie, porosité) qui assurent une régénération osseuse et une formation de l'os optimisées en un temps significativement réduit par rapport à un matériau de régénération osseuse tel que celui des documents WO2015/049336 et US2002/0114755.

En particulier, dans le cadre de la présente invention, il a été mis en évidence que, lorsque le ratio en poids défini entre ladite première phase solide d'hydroxyapatite d'origine naturelle et ladite deuxième phase synthétique solide de phosphate de calcium est compris entre 99/1 et 1/99, et est de préférence de 95/5, 90/10, 85/15, 80/20, 75/25, 70/30, 65/35, 60/40, 55/45, 50/50, 45/55, 40/60, 35/65, 30/70, 25/75, 20/80, 15/85, 10/90 ou 5/95, le temps de régénération osseuse est significativement réduit. Ceci est dû au fait qu'avec un tel ratio, au moins l'un des relargages en calcium et/ou en phosphore sous forme d'ions libres est tel que du calcium et/ou du phosphore se trouve alors dans le milieu environnant de régénération osseuse en une quantité permettant d'y augmenter significativement la concentration par rapport à la concentration naturellement rencontrée, alors que l'hydroxyapatite seule a tendance à capturer le calcium et le phosphore sous forme d'ions libres et à en dépléter le milieu environnent du site de régénération osseuse.

Notons que des matériaux de régénération osseuse bi-phasiques sont décrits dans l'état de la technique, en particulier des matériaux consistant en une association d'hydroxyapatite synthétique et de phosphate de calcium tricalcique synthétique : il s'agit donc de matériaux 100% synthétiques qui, comme pour un matériau de régénération tel qu'indiqué au début, imposent une durée de régénération osseuse relativement longue. Par ailleurs, de tels matériaux de régénération osseuse bi-phasiques totalement synthétiques ne permettent pas, au contraire d'un matériau selon l'invention, d'assurer une régénération osseuse optimale au site d'implantation en termes de prolifération et de différentiation des cellules osseuses car ils ne possèdent pas une topographie superficielle ni une microporosité adéquates.

De préférence, selon l'invention, ladite deuxième phase synthétique solide de phosphate de calcium présente un produit de solubilité Ks supérieur à celui de ladite première phase d'hydroxyapatite d'origine naturelle. Ceci est particulièrement avantageux puisqu'il convient d'apporter du calcium et du phosphore sous forme d'ions libres dans l'environnement direct du site d'implantation tout en conservant les propriétés structurelles (taille des pores, surface spécifique, ...) et de composition de la phase solide d'hydroxyapatite d'origine naturelle. Puisque la solubilité de la deuxième phase est supérieure, c'est essentiellement cette phase en particulier qui va être à l'origine de la libération de calcium et de phosphore sous forme d'ions libres (par exemple Ca²⁺ et PO₄³⁻) et non pas l'hydroxyapatite d'origine naturelle qui se dissout nettement moins et qui, au contraire, a tendance à fixer ces ions au départ de l'environnement d'implantation.

Avantageusement, selon l'invention, ladite première phase solide d'hydroxyapatite d'origine naturelle présente une surface spécifique supérieure à 4m²/g.

Préférentiellement, selon l'invention, ladite deuxième phase synthétique solide de phosphate de calcium est choisie dans le groupe constitué du phosphate de calcium monocalcique (MCP), du phosphate de calcium bicalcique (DCP), du phosphate octacalcique (OCP), de l'apatite déficiente en calcium (CDA), du phosphate de calcium amorphe (ACP), du phosphate de calcium tricalcique (TCP), du phosphate de calcium tétracalcique (TTCP), et leurs mélanges.

Avantageusement, selon l'invention, ladite première phase d'hydroxyapatite d'origine naturelle est de l'hydroxyapatite obtenue à partir d'un matériau osseux d'origine naturelle, en particulier à partir d'un matériau osseux d'origine animale. Par exemple, l'hydroxyapatite d'origine naturelle peut être de l'hydroxyapatite obtenue à partir d'un matériau osseux provenant d'un mammifère (bovin, cheval, cochon ou mouton), de corail ou de sèche.

De façon préférée, selon l'invention, ladite hydroxyapatite d'origine naturelle macroporeuse de ladite première phase est une hydroxyapatite d'origine naturelle macroporeuse au moins partiellement frittée. Le frittage contrôlé (chauffage d'une poudre sans la mener à la fusion) permet avantageusement d'obtenir une hydroxyapatite d'origine naturelle présentant des propriétés accrues de résistance mécanique tout en conservant une micro-porosité et une topographie de surface adéquates, ce qui permet de faciliter l'implantation et d'améliorer la stabilité à long terme après implantation.

Avantageusement, le matériau de régénération osseuse selon l'invention, comprend en outre au moins un agent thérapeutique choisi dans le groupe constitué des antibiotiques, des antiviraux, des antiinflammatoires, des hormones comme les stéroïdes, des facteurs de croissance comme les BMP (« Bone Morphogenetic Protein »), des agents anti-rejets, des cellules souches, et leurs mélanges.

De préférence, le matériau de régénération osseuse selon l'invention est destiné à être utilisé comme implant ou prothèse pour une formation d'os, une régénération d'os ou pour une réparation d'os chez un mammifère, de préférence chez un humain.

D'autres formes de réalisation d'un matériau de régénération osseuse suivant l'invention sont indiquées dans les revendications annexées.

L'invention a aussi pour objet un dispositif médical contenant un matériau de régénération osseuse selon l'invention.

D'autres formes de réalisation d'un dispositif médical suivant l'invention sont indiquées dans les revendications annexées.

L'invention a aussi pour objet une composition de régénération osseuse comprenant un matériau de régénération osseuse selon l'invention.

D'autres formes de réalisation d'une composition suivant l'invention sont indiquées dans les revendications annexées.

La présente invention porte également sur un procédé d'enrichissement d'un matériau de régénération osseuse selon l'invention, ledit procédé comprenant:
- une étape d'amenée d'un matériau de régénération osseuse, et
- une étape d'enrichissement dudit matériau de régénération osseuse, en calcium et en phosphore,
ledit procédé étant caractérisé en ce que ladite étape d'enrichissement en calcium et en phosphore est réalisée par au moins un premier et au moins un deuxième trempage distincts se succédant l'un l'autre dans un ordre quelconque, ledit au moins un premier trempage ayant lieu dans une première solution comprenant du calcium et ledit au moins un deuxième trempage ayant lieu dans une deuxième solution comprenant du phosphore.

De préférence, selon le procédé suivant l'invention, ledit premier trempage a lieu dans une première solution de Ca(NO₃)₂.4H₂O, de CaCl₂.2H₂O, de Ca(OH)₂, CaSO₄.2H₂O ou de CaCO₃.

Avantageusement, selon le procédé suivant l'invention, ledit deuxième trempage a lieu dans une deuxième solution de Na₃PO₄, de Na₂HPO₄, de NaH₂PO₄.H₂O, de K₃PO₄, de K₂HPO₄, de KH₂PO₄, de K₂HPO₄, de (NH₄)₃PO₄, de (NH₄)₂HPO₄ ou de NH₄H₂PO₄.

D'autres formes de réalisation d'un procédé suivant l'invention sont indiquées dans les revendications annexées.

D'autres caractéristiques, détails et avantages de l'invention ressortiront de la description donnée ci-après, à titre non limitatif et en faisant référence aux figures annexés.

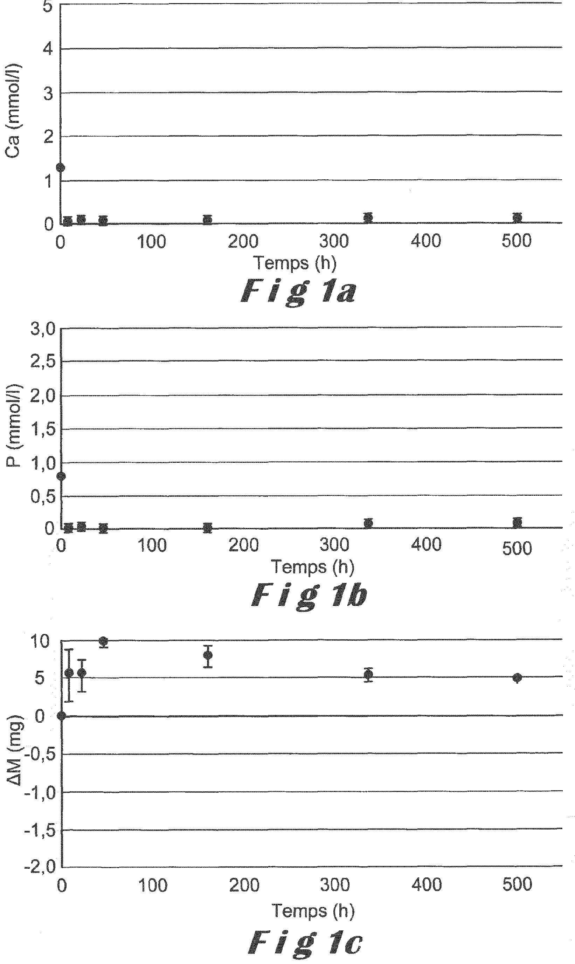

Les figures 1a, 1b et 1c illustrent respectivement l'évolution de la concentration en calcium (Ca) au cours du temps pour une phase solide d'hydroxyapatite d'origine naturelle bovine non enrichie immergée dans un milieu HBSS, l'évolution de la concentration en phosphore (P) au cours du temps pour une phase solide d'hydroxyapatite d'origine naturelle bovine non enrichie immergée dans un milieu HBSS et la prise de poids au cours du temps pour une phase solide d'hydroxyapatite d'origine naturelle bovine non enrichie immergée dans un milieu HBSS.

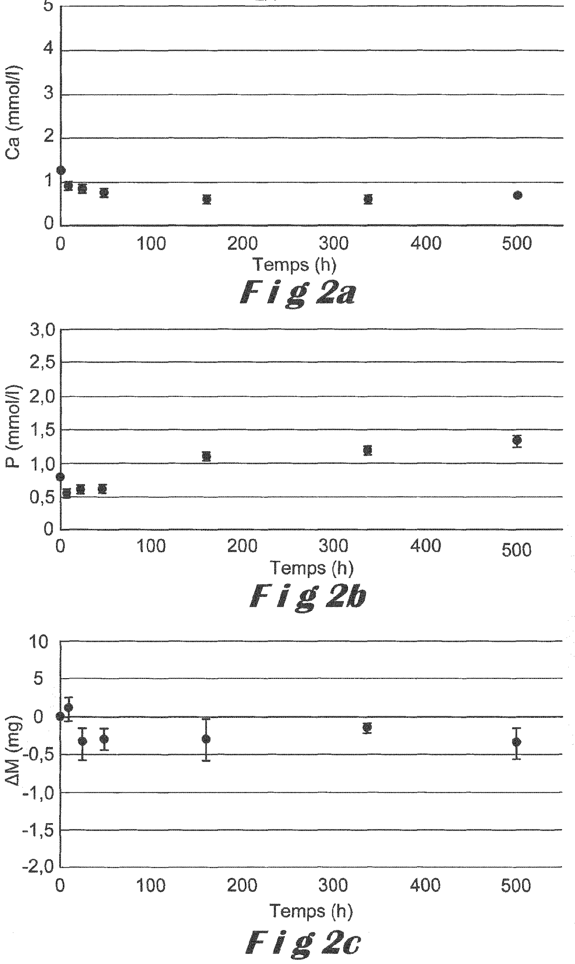

Les figures 2a, 2b, et 2c illustrent respectivement l'évolution de la concentration en calcium (Ca) au cours du temps pour un matériau immergé dans un milieu HBSS et comprenant une phase solide d'hydroxyapatite d'origine naturelle bovine enrichie par une phase synthétique solide de phosphate de calcium (ratio 90/10), l'évolution de la concentration en phosphore (P) au cours du temps pour un matériau immergé dans un milieu HBSS et comprenant une phase solide d'hydroxyapatite d'origine naturelle bovine enrichie par une phase synthétique solide de phosphate de calcium (ratio 90/10) et la prise de poids pour un matériau immergé dans un milieu HBSS et comprenant une phase solide d'hydroxyapatite d'origine naturelle bovine enrichie par une phase synthétique solide de phosphate de calcium (ratio 90/10).

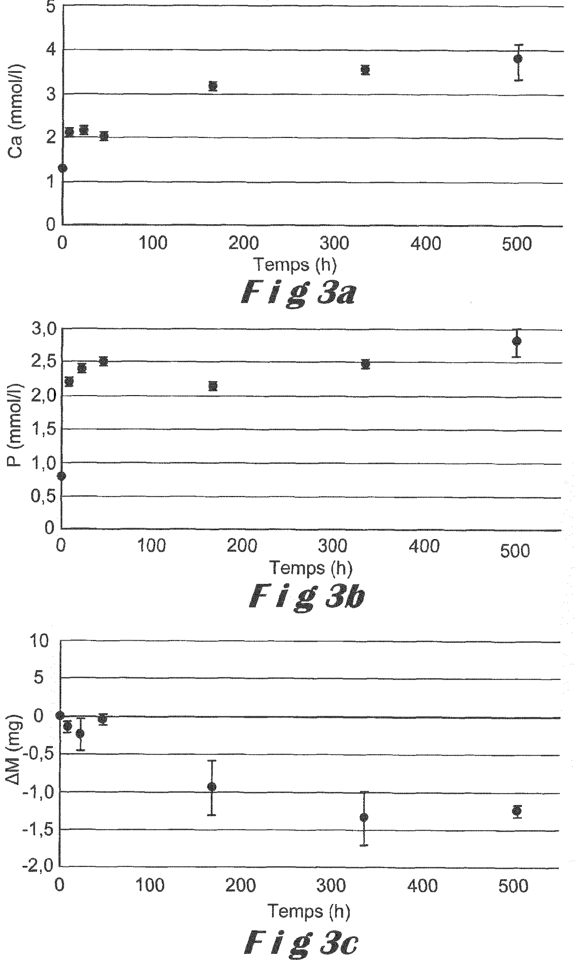

Les figures 3a, 3b, et 3c illustrent respectivement l'évolution de la concentration en calcium (Ca) au cours du temps pour un matériau immergé dans un milieu HBSS et comprenant une phase solide d'hydroxyapatite d'origine naturelle bovine enrichie par une phase synthétique solide de phosphate de calcium (ratio 80/20), l'évolution de la concentration en phosphore (P) au cours du temps pour un matériau immergé dans un milieu HBSS et comprenant une phase solide d'hydroxyapatite d'origine naturelle bovine enrichie par une phase synthétique solide de phosphate de calcium (ratio 80/20) et la prise de poids pour un matériau immergé dans un milieu HBSS et comprenant une phase solide d'hydroxyapatite d'origine naturelle bovine enrichie par une phase synthétique solide de phosphate de calcium (ratio 80/20).

### Exemples

### Comparaison des relargages en calcium et en phosphore sous forme d'ions libres au cours du temps pour différents matériaux de régénération osseuse - tests de solubilité

Des essais comparatifs ont été réalisés afin de déterminer les quantités (concentrations) en calcium et en phosphore relarguées au cours du temps (tests de solubilité) au départ de différents matériaux de régénération osseuse, ceci dans un milieu reproduisant les conditions in-vivo d'implantation.

### 1. Méthodologie

Pour ce faire, les taux et les quantités (concentrations) en calcium et en phosphore relargués ont été mesurés dans un milieu simulant les conditions d'implantation in-vivo (plasma sanguin humain), c'est-à-dire dans un milieu HBSS (Hank's balanced salt médium) présentant un pH de 7 et dont la composition est reprise au tableau 1 ci-dessous :

**Tableau 1**

| | Concentrations (mM) |
|---|---|
| Na⁺ | 142,8 |
| K⁺ | 5,8 |
| Mg²⁺ | 0,9 |
| Ca²⁺ | 1,3 |
| Cl⁻ | 146,8 |
| HCO³⁻ | 4,2 |
| HPO₄²⁻ | 0,3 |
| H₂PO⁴⁻ | 0,4 |
| SO₄²⁻ | 0,4 |
| Glucose | 5,6 |

Les matériaux de régénération osseuse suivants ont été testés :
- Matériau 1 : 0,5 g de phase solide d'hydroxyapatite d'origine naturelle bovine non enrichie présentant des pores d'une taille comprise entre 50 et 100 µm ;
- Matériau 2: 0,5 g de phase solide d'hydroxyapatite d'origine naturelle bovine présentant des pores d'une taille comprise entre 50 et 100 µm et enrichie par une phase synthétique solide de phosphate de calcium selon un ratio en poids phase solide d'hydroxyapatite/ phase synthétique solide de phosphate de calcium de 90/10, la phase de phosphate de calcium présentant un ratio molaire Ca/P inférieur à 1,67; et
- Matériau 3: 0,5g de phase solide d'hydroxyapatite d'origine naturelle bovine présentant des pores d'une taille comprise entre 50 et 100µm et enrichie par une phase synthétique solide de phosphate de calcium selon un ratio en poids phase solide d'hydroxyapatite/ phase synthétique solide de phosphate de calcium de 80/20, la phase de phosphate de calcium présentant un ratio molaire Ca/P inférieur à 1,67.

La phase solide d'hydroxyapatite d'origine naturelle bovine est obtenue selon le procédé décrit dans le document WO2015/049336 et est plus particulièrement composée de particules d'hydroxyapatite présentant une taille comprise entre 0,25 mm et 1 mm.

Les matériaux comprenant une première phase solide d'hydroxyapatite d'origine naturelle et une deuxième phase synthétique solide de phosphate de calcium ont été obtenus par trempages successifs et alternés d'une durée de 5 minutes de la première phase solide d'hydroxyapatite d'origine naturelle (particules d'hydroxyapatite) dans des bains distincts de Ca(NO₃)₂.4H₂O (1M, pH=10) et de NaH₂PO₄.H₂O (0,5M, pH=10). Le premier bain permet d'enrichir les particules d'hydroxyapatite d'origine naturelle en calcium (Ca²⁺), le second bain en phosphore (PO₄³⁻).

Suite à 4 trempages successifs (2 trempages dans chaque bain) pour obtenir un ratio en poids phase solide d'hydroxyapatite/ phase synthétique solide de phosphate de calcium de 90/10 ou suite à 6 trempages successifs (3 trempages dans chaque bain) pour obtenir un ratio en poids phase solide d'hydroxyapatite/ phase synthétique solide de phosphate de calcium de 80/20, les particules d'hydroxyapatite d'origine naturelle enrichies en calcium et en phosphore ont été rincées afin d'éliminer les excès de calcium et de phosphore puis séchées à une température de 100°C durant 6 heures.

Un total de 36 échantillons (de 0,5 g) ont été testés simultanément par immersion dans 10 ml de milieu HBSS à une température de 37°C. Des mesures de relargage en calcium et en phosphore sous forme d'ions libres ont été réalisées après 8h, 24h, 48h, 1 semaine, 2 semaines et 3 semaines sur deux échantillons pour chaque matériau et par durée, ces échantillons étant rincés et séchés à une température de 100°C après chacune de ces durées.

Les quantités (concentrations) en calcium et en phosphore relarguées au départ de chaque échantillon dans le milieu HBSS ont été mesurées selon la technique ICP-AES (Inductively Coupled Plasma - Atomic Emission Spectroscopy), ceci suite à une élimination des éventuelles matières en suspension dans le milieu HBSS par centrifugation. Le poids des matériaux ont été définis par pesées (gravimétrie) avant et après immersion dans le milieu HBSS, ceci pour chacune des durées mentionnées ci-dessus.

### 2. Résultats

Les résultats obtenus sont illustrés aux figures 1 à 3. Pour chacun des matériaux (échantillons), les concentrations de départ en calcium et en phosphore sont respectivement de 1,26 mmol/l et de 0,78 mmol/l, ce qui correspond aux concentrations en ces ions dans le milieu HBSS initial.

Ces résultats montrent que, lorsque le matériau 1 - phase solide d'hydroxyapatite d'origine naturelle bovine non enrichie - est immergé dans le milieu HBSS, les concentrations en calcium et en phosphore sous forme d'ions libres décroissent avant de se stabiliser (figures 1a et 1b). En parallèle, une augmentation en poids du matériau 1 est observée (figure 1c). Par exemple, après 3 semaines, la concentration en calcium dans le milieu est de 0,06 mmol/l tandis que la concentration en phosphore dans le milieu est de 0,03 mmol/l pour un gain de poids moyen par échantillon de 5 mg.

Pour le matériau 2 - phase solide d'hydroxyapatite d'origine naturelle bovine enrichie par une phase synthétique solide de phosphate de calcium (ratio 90/10) - les concentrations en calcium et en phosphore commencent par décroitre avec une rapide stabilisation de la concentration en calcium (0,6 mmol/l) tandis que la concentration en phosphore augmente progressivement jusqu'à atteindre une valeur finale de 1,34 mmol/l après 3 semaines d'immersion (figures 2a et 2b), cette concentration étant supérieure à la concentration initiale en phosphore dans le milieu HBSS. Les poids des échantillons du matériau 2 ont d'abord quelque peu augmenté avant de décroitre jusqu'à une perte en poids de 3,5 mg (figure 2c).

Concernant le matériau 3 - phase solide d'hydroxyapatite d'origine naturelle bovine enrichie par une phase synthétique solide de phosphate de calcium (ratio 80/20) -, les concentrations en calcium et en phosphore augmentent au cours du temps tandis que le poids des échantillons décroit (figures 3a à 3c). Les concentrations finales en calcium et en phosphore, après 3 semaines d'immersion dans le milieu HBSS, sont respectivement de 3,69 mmol/l et de 2,78 mmol/l pour une perte en poids de 12,5 mg, ces concentrations étant supérieures à celles initialement mesurées dans le milieu HBSS.

### 3. Conclusion

Les résultats obtenus avec le matériau 1 - phase solide d'hydroxyapatite d'origine naturelle bovine non enrichie - soulignent la capacité de l'hydroxyapatite à fixer le calcium et le phosphore sous forme d'ions libres présents dans le milieu HBSS pour former une couche d'apatite à sa surface. Déjà après 8h d'immersion, la majeure partie des ions libres de calcium et de phosphore sont absorbés à la surface des échantillons. Ces résultats concordent avec les relevés gravimétriques qui indiquent que les échantillons sont plus lourds après 3 semaines d'immersion. Ces résultats indiquent donc que le matériau 1 (hydroxyapatite non enrichie) fixe le calcium et le phosphore sous forme d'ions libres et que la dissolution de l'hydroxyapatite dans le milieu HBSS est négligeable.

Ces résultats font ressortir, pour le matériau 2 - phase solide d'hydroxyapatite d'origine naturelle bovine enrichie par une phase synthétique solide de phosphate de calcium (ratio 90/10) - que deux phénomènes ont lieu de façon concomitante puisque les concentrations en calcium et en phosphore décroissent d'abord toutes deux, ce qui est à nouveau lié à la capacité que possède l'hydroxyapatite de fixer ces ions. Toutefois, la concentration en phosphore dans le milieu augmente ensuite et dépasse même la concentration en ce composé dans le milieu HBSS initial alors que la concentration en calcium reste inférieure à celle initialement mesurée dans le milieu HBSS en étant cependant supérieure à celle mesurée lorsque le matériau 1 est immergé dans ce même milieu. En parallèle, après 3 semaines, les résultats gravimétriques montrent que les échantillons ont perdu du poids. L'ensemble de ces résultats pour le matériau 2 indiquent que ce matériau se dissout dans le milieu HBSS, ce qui ne peut être attribué qu'à la phase d'enrichissement (phase synthétique solide de phosphate de calcium) puisque les résultats obtenus avec le matériau 1 montrent que la dissolution de l'hydroxyapatite dans les mêmes conditions est totalement négligeable.

Les résultats obtenus avec le matériau 3 - phase solide d'hydroxyapatite d'origine naturelle bovine enrichie par une phase synthétique solide de phosphate de calcium (ratio 80/20) - démontrent qu'aucune diminution des concentrations en calcium et en phosphore n'a lieu, que du contraire puisque les concentrations en calcium et en phosphore augmentent au cours du temps en parallèle avec une perte de poids des échantillons. De ces observations, il peut être conclu que ces augmentations des concentrations en calcium et en phosphore sont dues à une dissolution de la deuxième phase du matériau 3 dans le milieu HBSS puisque les résultats obtenus avec le matériau 1 montrent que la dissolution de l'hydroxyapatite dans les mêmes conditions est totalement négligeable. Par ailleurs, il peut être observé que le matériau 3 ne fixe pas le calcium et le phosphore sous forme d'ions libres relargués ou seulement de façon négligeable, ce qui assure que ces derniers restent en solution.

L'ensemble de ces résultats montre aussi que l'enrichissement (phase synthétique solide de phosphate de calcium) est plus soluble que l'hydroxyapatite et qu'il est en mesure de relarguer du calcium et du phosphore sous forme d'ions libres (Ca²⁺ et PO₄³⁻) dans le milieu environnement (HBSS mimant le plasma sanguin).

## Revendications

1. Matériau de régénération osseuse comprenant une première phase solide d'hydroxyapatite d'origine naturelle macroporeuse présentant des pores de diamètres supérieurs ou égaux à 50 µm, de préférence des pores de diamètres compris entre 50 et 100 µm, ledit matériau de régénération osseuse étant **caractérisé en ce que** ladite première phase solide d'hydroxyapatite est enrichie par une deuxième phase de phosphate de calcium, ladite deuxième phase étant une phase synthétique solide, présentant un ratio molaire Ca/P compris entre 0,2 et 2, de préférence compris entre 0,3 et 1,8, préférentiellement compris entre 0,5 et 1,65, et présentant un produit de solubilité Ks supérieur à celui de ladite première phase d'hydroxyapatite d'origine naturelle, ledit matériau de régénération osseuse présentant un ratio en poids défini entre ladite première phase solide d'hydroxyapatite d'origine naturelle et ladite deuxième phase synthétique solide de phosphate de calcium compris entre 99/1 et 1/99.

2. Matériau de régénération osseuse selon la revendication 1, **caractérisé en ce que** ladite première phase solide d'hydroxyapatite d'origine naturelle présente une surface spécifique supérieure à 4m²/g.

3. Matériau de régénération osseuse selon l'une quelconque des revendications précédentes, **caractérisé en ce que** ladite deuxième phase synthétique solide de phosphate de calcium est choisie dans le groupe constitué du phosphate de calcium monocalcique (MCP), du phosphate de calcium bicalcique (DCP), du phosphate octacalcique (OCP), de l'apatite déficiente en calcium (CDA), du phosphate de calcium amorphe (ACP), du phosphate de calcium tricalcique (TCP), du phosphate de calcium tétracalcique (TTCP), et leurs mélanges.

4. Matériau de régénération osseuse selon l'une quelconque des revendications précédentes, dans lequel ladite première phase d'hydroxyapatite d'origine naturelle est de l'hydroxyapatite obtenue à partir d'un matériau osseux d'origine naturelle, en particulier à partir d'un matériau osseux d'origine animale.

5. Matériau de régénération osseuse selon l'une quelconque des revendications précédentes, dans lequel ladite hydroxyapatite d'origine naturelle macroporeuse de ladite première phase est une hydroxyapatite d'origine naturelle macroporeuse au moins partiellement frittée.

6. Matériau de régénération osseuse selon l'une quelconque des revendications précédentes, comprenant en outre au moins un agent thérapeutique choisi dans le groupe constitué des antibiotiques, des antiviraux, des antiinflammatoires, des hormones comme les stéroïdes, des facteurs de croissance comme les BMP (« Bone Morphogenetic Protein »), des agents anti-rejets, des cellules souches, et leurs mélanges.

7. Matériau de régénération osseuse selon l'une quelconque des revendications précédentes, destiné à être utilisé comme implant ou prothèse pour une formation d'os, une régénération d'os ou pour une réparation d'os chez un mammifère, de préférence chez un humain.

8. Dispositif médical contenant un matériau de régénération osseuse selon l'une quelconque des revendications 1 à 6.

9. Composition de régénération osseuse comprenant un matériau de régénération osseuse selon l'une quelconque des revendications 1 à 6.

10. Procédé d'enrichissement d'un matériau de régénération osseuse selon l'une quelconque des revendications 1 à 6, ledit procédé comprenant:
- une étape d'amenée d'un matériau de régénération osseuse, et
- une étape d'enrichissement dudit matériau de régénération osseuse, en calcium et en phosphore,
ledit procédé étant **caractérisé en ce que** ladite étape d'enrichissement en calcium et en phosphore est réalisée par au moins un premier et au moins un deuxième trempage distincts se succédant l'un l'autre dans un ordre quelconque, ledit au moins un premier trempage ayant lieu dans une première solution comprenant du calcium et ledit au moins un deuxième trempage ayant lieu dans une deuxième solution comprenant du phosphore.

11. Procédé d'enrichissement d'un matériau de régénération osseuse selon la revendication 10, **caractérisé en ce que** ledit premier trempage a lieu dans une première solution de Ca(NO₃)₂.4H₂O, de CaCl₂.2H₂O, de Ca(OH)₂, CaSO₄.2H₂O ou de CaCO₃.

12. Procédé d'enrichissement d'un matériau de régénération osseuse selon la revendication 10 ou 11, **caractérisé en ce que** ledit deuxième trempage a lieu dans une deuxième solution de Na₃PO₄, de Na₂HPO₄, de NaH₂PO₄.H₂O, de K₃PO₄, de K₂HPO₄, de KH₂PO₄, de
K₂HPO₄, de (NH₄)₃PO₄, de (NH₄)₂HPO₄ ou de NH₄H₂PO₄.

## Patentansprüche

1. Material zur Knochenregeneration, umfassend eine erste feste Phase von Hydroxyapatit makroporösen, natürlichen Ursprungs, das Poren mit Durchmessern von mehr als oder gleich 50 µm, vorzugsweise Poren mit Durchmessern zwischen 50 und 100 µm, vorweist, wobei das Material zur Knochenregeneration **dadurch gekennzeichnet ist, dass** die erste feste Phase von Hydroxyapatit durch eine zweite Phase von Calciumphosphat angereichert ist, wobei die zweite Phase eine zweite feste synthetische Phase ist, die ein Molverhältnis Ca / P zwischen 0,2 und 2, vorzugsweise zwischen 0,3 und 1,8, bevorzugt zwischen 0,5 und 1,65, vorweist und ein Produkt mit der Löslichkeit Ks vorweist, die höher als diejenige der ersten Phase von Hydroxyapatit natürlichen Ursprungs ist, wobei das Material zur Knochenregeneration ein Gewichtsverhältnis, das zwischen der ersten festen Phase von Hydroxyapatit natürlichen Ursprungs und der zweiten festen Phase von synthetischen Calciumphosphat definiert ist, zwischen 99/1 und 1/99 vorweist.

2. Material zur Knochenregeneration nach Anspruch 1, **dadurch gekennzeichnet, dass** die erste feste Phase von Hydroxyapatit natürlichen Ursprungs eine spezifische Oberfläche von mehr als 4 m² / g vorweist.

3. Material zur Knochenregeneration nach einem der vorhergehenden Ansprüche, **dadurch gekennzeichnet, dass** die zweite feste, synthetische Phase von Calciumphosphat aus der Gruppe ausgewählt ist, bestehend aus Monocalciumphosphat (MCP), Dicalciumphosphat (DCP), Octacalciumphosphat (OCP), Apatit mit Calciummangel (CDA), amorphem Calciumphosphat (ACP), Tricalciumphosphat (TCP), Tetracalciumphosphat (TTCP) und Gemischen davon.

4. Material zur Knochenregeneration nach einem der vorhergehenden Ansprüche, wobei die erste Phase von Hydroxyapatit natürlichen Ursprungs Hydroxyapatit ist, das aus einem Knochenmaterial natürlichen Ursprungs, insbesondere aus einem Knochenmaterial tierischen Ursprungs, erhalten wurde.

5. Material zur Knochenregeneration nach einem der vorhergehenden Ansprüche, wobei der makroporöse Hydroxyapatit natürlichen Ursprungs der ersten Phase ein zumindest teilweise gesinterter, makroporöser Hydroxyapatit natürlichen Ursprungs ist.

6. Material zur Knochenregeneration nach einem der vorhergehenden Ansprüche, ferner umfassend mindestens ein therapeutisches Mittel, das aus der Gruppe ausgewählt ist, bestehend aus Antibiotika, Antivirusmittel, entzündungshemmende Mittel, Hormone wie Steroide, Wachstumsfaktoren wie BMPs ("Bone Morphogenetic Protein" ), Antiabstoßungsmittel, Stammzellen und Gemischen davon.

7. Material zur Knochenregeneration nach einem vorhergehenden Anspruch zur Verwendung als Implantat oder Prothese zur Knochenbildung, Knochenregeneration oder zur Knochenreparatur bei einem Säugetier, vorzugsweise bei einem Menschen.

8. Medizinische Vorrichtung, die ein Material zur Knochenregeneration nach einem der Ansprüche 1 bis 6 enthält.

9. Zusammensetzung zur Knochenregeneration, umfassend ein Material zur Knochenregeneration nach einem der Ansprüche 1 bis 6.

10. Verfahren zur Anreicherung eines Materials zur Knochenregeneration nach einem der Ansprüche 1 bis 6, wobei das Verfahren umfasst:
- einen Schritt der Zufuhr eines Materials zur Knochenregeneration und
- einen Schritt der Anreicherung des Materials zur Knochenregeneration mit Calcium und Phosphor;
wobei das Verfahren **dadurch gekennzeichnet ist, dass** der Schritt der Anreicherung mit Calcium und Phosphor durch mindestens ein erstes Einweichen und mindestens ein zweites unterschiedliches Einweichen durchgeführt wird, die aufeinander in beliebiger Reihenfolge folgen, wobei das mindestens eine erste Einweichen in einer ersten Lösung, die Calcium umfasst, stattfindet, und wobei das mindestens eine zweite Einweichen in einer zweiten Lösung, die Phosphor umfasst, stattfindet.

11. Verfahren zur Anreicherung eines Material zur Knochenregeneration nach Anspruch 10, **dadurch gekennzeichnet, dass** das erste Einweichen in einer ersten Lösung von Ca(NO₃)₂.4H₂O, CaCl₂.2H₂O, Ca(OH)₂, CaSO₄.2H2O oder CaCO₃ stattfindet.

12. Verfahren zur Anreicherung eines Materials zur Knochenregeneration nach Anspruch 10 oder 11, **dadurch gekennzeichnet, dass** das zweite Einweichen in einer zweiten Lösung von Na₃PO₄, Na₂HPO₄, NaH₂PO₄, H₂O, K₃PO₄, K₂HPO₄, KH₂PO₄, K₂HPO₄, (NH₄)₃PO₄, (NH₄)₂HPO₄ oder NH₄H₂PO₄ stattfindet.

## Claims

1. Bone regeneration material comprising a first solid phase of macroporous hydroxyapatite of natural origin having pores larger than or equal to 50µm in diameter, preferably pores between 50 and 100µm in diameter, said bone regeneration material being **characterised in that** said first solid phase of hydroxyapatite is enriched by a second phase of calcium phosphate, said second phase being a solid synthetic phase, having a Ca/P molar ratio of between 0.2 and 2, preferably between 0.3 and 1.8, preferentially between 0.5 and 1.65, and having solubility product Ks greater than that of said first phase of hydroxyapatite of natural origin, said bone regeneration material having a defined weight ratio between said first solid phase of hydroxyapatite of natural origin and said second solid synthetic phase of calcium phosphate of between 99:1 and 1:99.

2. Bone regeneration material according to claim 1, **characterised in that** said first solid phase of hydroxyapatite of natural origin has a specific surface area larger than 4m²/g.

3. Bone regeneration material according to any one of the preceding claims, **characterised in that** said second solid synthetic phase of calcium phosphate is selected from the group consisting of monocalcium phosphate (MCP), dicalcium phosphate (DCP), octacalcium phosphate (OCP), calcium deficient apatite (CDA), amorphous calcium phosphate (ACP), tricalcium phosphate (TCP), tetracalcium phosphate (TTCP) and mixtures thereof.

4. Bone regeneration material according to any one of the preceding claims, wherein said first phase of hydroxyapatite of natural origin is hydroxyapatite obtained from bone material of natural origin, in particular from bone material of animal origin.

5. Bone regeneration material according to any one of the preceding claims, wherein said macroporous hydroxyapatite of natural origin of said first phase is a macroporous hydroxyapatite of natural origin, at least partially sintered.

6. Bone regeneration material according to any one of the preceding claims, further comprising at least one therapeutic agent selected from the group consisting of antibiotics, antivirals, antiinflammatories, hormones such as steroids, growth factors such as BMPs ('Bone Morphogenetic Protein'), anti-rejection agents, stem cells and mixtures thereof.

7. Bone regeneration material according to any one of the preceding claims, for use as an implant or prosthesis for bone formation, bone regeneration or bone repair in a mammal, preferably in a human.

8. Medical device containing a bone regeneration material according to any one of claims 1 to 6.

9. Bone regeneration composition comprising a bone regeneration material according to any one of claims 1 to 6.

10. Method for enriching a bone regeneration material according to any one of claims 1 to 6, said method comprising:
- a stage of supplying a bone regeneration material, and
- a stage of enriching said bone regeneration material with calcium and phosphorus,
said method being **characterised in that** said stage of enriching with calcium and phosphorus is carried out by at least one first and at least one second separate soak, one following the other in any order, said at least one first soak taking place in a first solution comprising calcium and said at least one second soak taking place in a second solution comprising phosphorus.

11. Method for enriching a bone regeneration material according to claim 10, **characterised in that** said first soak takes place in a first solution of Ca(NO₃)₂.4H₂O, CaCl₂.2H₂O, Ca(OH)₂, CaSO₄.2H₂O or CaCO₃.

12. Method for enriching a bone regeneration material according to claim 10 or 11, **characterised in that** said second soak takes place in a second solution of Na₃PO₄, Na₂HPO₄, NaH₂PO₄.H₂O, K₃PO₄, K₂HPO₄, KH₂PO₄, K₂HPO₄, (NH₄)₃PO₄, (NH₄)₂HPO₄ or NH₄H₂PO₄.
